(19) 

Europäisches
Patentamt
European
Patent Office
Office européen
des brevets

(11) **EP 4 068 304 A1**

(12) **EUROPEAN PATENT APPLICATION**
published in accordance with Art. 153(4) EPC

(43) Date of publication:
**05.10.2022 Bulletin 2022/40**

(21) Application number: **20891415.0**

(22) Date of filing: **25.11.2020**

(51) International Patent Classification (IPC):
***G16H 50/30*** (2018.01)

(52) Cooperative Patent Classification (CPC):
**G16H 50/30; G06F 3/0481; G16H 10/65**

(86) International application number:
**PCT/CN2020/131332**

(87) International publication number:
**WO 2021/104282 (03.06.2021 Gazette 2021/22)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.11.2019 CN 201911167179**

(71) Applicant: BOE Technology Group Co., Ltd.
**Beijing 100015 (CN)**

(72) Inventors:
• **TONG, Xiaotong**
  **Beijing 100176 (CN)**
• **ZHANG, Xu**
  **Beijing 100176 (CN)**
• **HU, Yanyang**
  **Beijing 100176 (CN)**
• **WANG, Hongliang**
  **Beijing 100176 (CN)**

(74) Representative: **Haseltine Lake Kempner LLP**
**Cheapside House**
**138 Cheapside**
**London EC2V 6BJ (GB)**

(54) **DATA DISPLAY METHOD AND APPARATUS, TERMINAL, AND STORAGE MEDIUM**

(57)     Disclosed are a method and an apparatus for displaying data, and a terminal and a storage medium thereof, which belong to the field of computer technologies. The method includes: displaying a first state diagram, wherein the first state diagram indicates a vital state of a living individual (S110); receiving items to be assessed, wherein the items to be assessed are intended to assess the vital state of the living individual (S 120); and displaying a second state diagram, wherein the second state diagram is a result of updating the first state diagram based on the items to be assessed (S130).

FIG. 1

**Description**

This application claims the priority to Chinese patent application No. 201911167179.9, filed on November 25, 2019 and entitled "METHOD AND APPARATUS FOR DISPLAYING DATA, AND DEVICE AND STORAGE MEDIUM THEREOF," the disclosure of which is incorporated herein by reference in its entirety.

**TECHNICAL FIELD**

The present disclosure relates to the field of computer technologies, and in particular, relates to a method and an apparatus for displaying data, and a terminal and a storage medium thereof.

**BACKGROUND**

With the development of computer technologies and medical technologies, users have an increasingly urgent demand for convenient knowing of their own health states to monitor the health states. A health state of a living individual can be monitored by acquiring data of multiple aspects reflecting the health state of the living individual and assessing the health state of the living individual based on the data of the multiple aspects.

**SUMMARY**

The present disclosure provides a method and an apparatus for displaying data, and a terminal and a storage medium thereof.

In a first aspect of the present disclosure, a method for displaying data is provided. The method includes: displaying a first state diagram, wherein the first state diagram indicates a vital state of a living individual; receiving items to be assessed, wherein the items to be assessed are intended to assess the vital state of the living individual; and displaying a second state diagram, wherein the second state diagram is a result of updating the first state diagram based on the items to be assessed.

Optionally, the second state diagram is a result of growing the first state diagram based on an integrity degree of the items to be assessed.

Optionally, the second state diagram is a result of assessing the vital state of the living individual based on the items to be assessed and adjusting a color of the first state diagram based on an assessment result of the vital state of the living individual.

Optionally, the second state diagram is a result of adding a first icon onto the first state diagram, wherein the first icon indicates an association relationship between the living individual and a specified defect, and the association relationship is acquired based on analysis of the items to be assessed.

Optionally, the method further includes: receiving a select operation of the first icon; and displaying analysis of the association relationship between the living individual and the specified defect based on the select operation of the first icon.

Optionally, the second state diagram is a result of adding a second icon onto the first state diagram, wherein the second icon indicates a processing assessment value of an operation performed by a user.

Optionally, a redemption icon is displayed in the second state diagram; and in response to displaying the second state diagram, the method further includes: receiving a select operation of the redemption icon; displaying a redemption page of articles to be redeemed by using the processing assessment value based on the select operation of the redemption icon; receiving a select operation on the redemption page; and performing an operation of redeeming an article indicated by the select operation on the redemption page by using the processing assessment value of the user, based on the select operation on the redemption page.

Optionally, an icon for prompting to complete the items to be assessed is further displayed on a display interface of at least one of the first state diagram and the second state diagram.

Optionally, an icon for prompting to view interaction knowledge related to the items to be assessed is further displayed on the display interface of at least one of the first state diagram and the second state diagram.

Optionally, an assessment icon is displayed on the display interface of at least one of the first state diagram and the second state diagram; and the method further includes: receiving a select operation of the assessment icon; acquiring a score for assessing the vital state of the living individual based on the items to be assessed with instruction of the select operation; and displaying a third icon on the display interface of the at least one state diagram, wherein the third icon interprets the score.

Optionally, the method further includes: receiving a select operation of the score; and displaying score details of the score based on the select operation of the score.

Optionally, prior to displaying the second state diagram, the method further includes: assessing the vital state of the living individual based on the items to be assessed; and the displaying the second state diagram includes: displaying the second state diagram based on an assessment result of the vital state of the living individual.

Optionally, prior to displaying the second state diagram, the method further includes: sending the items to be assessed to a server; and receiving an assessment result of the vital state of the living individual sent by the server, wherein the assessment result is acquired based on the items to be assessed; and the displaying the second state diagram includes: displaying the second state diagram based on the assessment result of the vital state of the living individual.

Optionally, both the first state diagram and the second state diagram are tree structure diagrams.

Optionally, the vital state of the living individual includes a health state of the user.

In a second aspect, an apparatus for displaying data is provided according to embodiments of the present disclosure. The apparatus includes: a displaying module, configured to display a first state diagram, wherein the first state diagram indicates a vital state of a living individual; and a receiving module, configured to receive items to be assessed, wherein the items to be assessed are intended to assess the vital state of the living individual; and the displaying module is further configured to display a second state diagram, wherein the second state diagram is a result of updating the first state diagram based on the items to be assessed.

Optionally, the second state diagram is a result of growing the first state diagram based on an integrity degree of the items to be assessed.

Optionally, the second state diagram is a result of assessing the vital state of the living individual based on the items to be assessed and adjusting a color of the first state diagram based on an assessment result of the vital state of the living individual.

Optionally, the second state diagram is a result of adding a first icon onto the first state diagram, wherein the first icon indicates an association relationship between the living individual and a specified defect, and the association relationship is acquired based on analysis of the items to be assessed.

Optionally, the receiving module is further configured to receive a select operation of the first icon; and the displaying module is further configured to display analysis of the association relationship between the living individual and the specified defect based on the select operation of the first icon.

Optionally, the second state diagram is a result of adding a second icon onto the first state diagram, wherein the second icon indicates a processing assessment value of an operation performed by a user.

Optionally, a redemption icon is displayed in the second state diagram; the receiving module is further configured to receive a select operation of the redemption icon; the displaying module is further configured to display a redemption page of articles to be redeemed by using the processing assessment value based on the select operation of the redemption icon; the receiving module is further configured to receive a select operation on the redemption page; and the apparatus further includes: a processing module, configured to perform an operation of redeeming an article indicated by the select operation on the redemption page by using the processing assessment value of the user, based on the select operation on the redemption page.

Optionally, an icon for prompting to complete the items to be assessed is further displayed on a display interface of at least one of the first state diagram and the second state diagram.

Optionally, an icon for prompting to view interaction knowledge related to the items to be assessed is further displayed on the display interface of at least one of the first state diagram and the second state diagram.

Optionally, an assessment icon is displayed on the display interface of at least one of the first state diagram and the second state diagram, and the receiving module is further configured to receive a select operation of the assessment icon; the processing module is further configured to acquire a score for assessing the vital state of the living individual based on the items to be assessed with instruction of the select operation; and the displaying module is further configured to display a third icon on the display interface of the at least one state diagram, wherein the third icon interprets the score.

Optionally, the receiving module is further configured to receive a select operation of the score; and the displaying module is further configured to display score details of the score based on the select operation of the score.

Optionally, the processing module is further configured to assess the vital state of the living individual based on the items to be assessed; and the displaying module is specifically configured to display the second state diagram based on the assessment result of the vital state of the living individual.

Optionally, the apparatus further includes: a sending module, configured to send the items to be assessed to a server; the receiving module is further configured to receive an assessment result of the vital state of the living individual sent by the server, wherein the assessment result is acquired based on the items to be assessed; and the displaying module is specifically configured to display the second state diagram based on the assessment result of the vital state of the living individual.

Optionally, both the first state diagram and the second state diagram are tree structure diagrams.

Optionally, the vital state of the living individual includes a health state of the user.

In a third aspect, a terminal is provided according to the embodiments of the present disclosure. The terminal

**EP 4 068 304 A1**

includes a memory, a processor, and a computer program stored on the memory and capable of being run by the processor, wherein the program, when run by the processor, causes the processor to perform the method for displaying data as described in the first aspect.

In a fourth aspect, a computer-readable storage medium storing a computer program therein is provided according to the embodiments of the present disclosure, wherein the computer program is run to perform the method for displaying data as described in the first aspect.

**BRIEF DESCRIPTION OF THE DRAWINGS**

FIG. 1 is a schematic flowchart of a method for displaying data according to an embodiment of the present disclosure;
FIG. 2 is a schematic diagram of a first state diagram according to an embodiment of the present disclosure;
FIG. 3 is a schematic display diagram of a second operation interface according to an embodiment of the present disclosure;
FIG. 4 is a schematic display diagram of a second operation interface according to another embodiment of the present disclosure;
FIG. 5 is a schematic diagram showing growth change of a second state diagram relative to a first state diagram according to an embodiment of the present disclosure;
FIG. 6 is a schematic diagram showing color change of the second state diagram relative to the first state diagram according to an embodiment of the present disclosure;
FIG. 7 is a schematic diagram of a first operation interface according to an embodiment of the present disclosure;
FIG. 8 is a schematic diagram showing score details according to an embodiment of the present disclosure;
FIG. 9 is a schematic diagram of a first operation interface according to another embodiment of the present disclosure;
FIG. 10 is a schematic diagram of an assessment result according to an embodiment of the present disclosure;
FIG. 11 is a flowchart of a method for estimating and judging according to an embodiment of the present disclosure;
FIG. 12 is a flowchart of another method for estimating and judging according to an embodiment of the present disclosure;
FIG. 13 is a flowchart of yet another method for estimating and judging according to an embodiment of the present disclosure;
FIG. 14 is a schematic interface diagram for explaining points according to an embodiment of the present disclosure;
FIG. 15 is a schematic diagram of a points redemption interface according to an embodiment of the present disclosure;
FIG. 16 is a schematic structure diagram of an apparatus for displaying data according to an embodiment of the present disclosure;
FIG. 17 is a schematic structure diagram of another apparatus for displaying data according to an embodiment of the present disclosure;
FIG. 18 is a schematic structure diagram of yet another apparatus for displaying data according to an embodiment of the present disclosure; and
FIG. 19 is a schematic structure diagram of a terminal according to an embodiment of the present disclosure.

**DETAILED DESCRIPTION**

The present disclosure will be further described in detail hereinafter in combination with the accompanying drawings and the embodiments. It can be understood that the specific embodiments described herein are only configured to explain content related to the present disclosure, but not to limit the present disclosure. In addition, it should be noted that for convenience of description, only parts related to the present disclosure are shown in the accompanying drawings.

It should be noted that the embodiments in the present disclosure and the items in the embodiments may be combined with each other without conflict. The present disclosure will be described in detail hereinafter with reference to the accompanying drawings and in combination with the embodiments.

At present, in case of displaying a health state of a living individual, only data reflecting the health state of the living individual is displayed. As a result, the living individual is unable to directly perceive his/her actual health state, adversely affecting the user experience.

In the embodiments of the present disclosure, a vital state of a living individual can be assessed from multiple dimensions by utilizing items to be assessed of the living individual, and can be displayed to a user in a graphic fashion, such that the user can visually know the vital state of the living individual. Furthermore, by acquiring the items to be assessed of the living individual, a state diagram for indicating the vital state of the living individual is updated, such that a display mode of the state diagram is enriched, enabling the vital state of the living individual to be displayed visually and effectively.

In an embodiment, a method for displaying data according to the embodiment of the present disclosure may be performed with a system architecture of a terminal and a server. That is, a system capable of transmitting data may be provided, and may include the server and the terminal. The terminal may access the server over a wireless network and send items to be assessed of a living individual to the server. The server may assess a vital state of the living individual based on the items to be assessed, and send an assessment result to the terminal, such that the terminal can display the vital state of the living individual based on the assessment result.

Optionally, the server may store multiple algorithm rules for assessing the vital state of the living individual, and may assess the vital state of the living individual based on the items to be assessed according to the algorithm rule. For example, the server may store an assessment algorithm for assessing an association relationship between the living individual and various defect indicators, such as an assessment algorithm for assessing whether the living individual suffering from hypertension, an assessment algorithm for assessing whether the living individual suffering from intermittent claudication, and an assessment algorithm for assessing whether the living individual suffering from common diseases like diabetes. Furthermore, the server may also store a comprehensive assessment algorithm. That is, in response to acquiring the items to be assessed of the living individual, a current comprehensive state of the living individual may be determined by comprehensive analysis based on all the items to be assessed. In addition, the server may also store all kinds of information of the user, such as account information, points information and historical data, and the server may provide the information to the terminal. Optionally, the server may be achieved by a server or a server cluster including a plurality of servers, which is not specifically limited in the embodiments of the present disclosure.

The terminal may be a mobile terminal or a computer. The terminal may display a specified operation interface, and the user may input the items to be assessed on the operation interface, such that the terminal can display a state diagram on the operation interface based on the items to be assessed, thereby displaying the vital state of the living individual visually and effectively. An application program, namely, a client, may be run in the terminal. At this time, the specified operation interface is an operation interface of the client. The user may input the items to be assessed on the operation interface of the client after registering and logging in an account at the client; or, in the specified operation interface, the items to be assessed may also be input on a webpage for displaying the vital state of the living individual.

In another embodiment, a method for displaying data according to the embodiment of the present disclosure may be performed by a terminal. In this embodiment, the terminal may receive items to be assessed of a living individual input by a user; a vital state of the living individual is assessed based on the items to be assessed; and a state diagram is displayed in the terminal based on an assessment result.

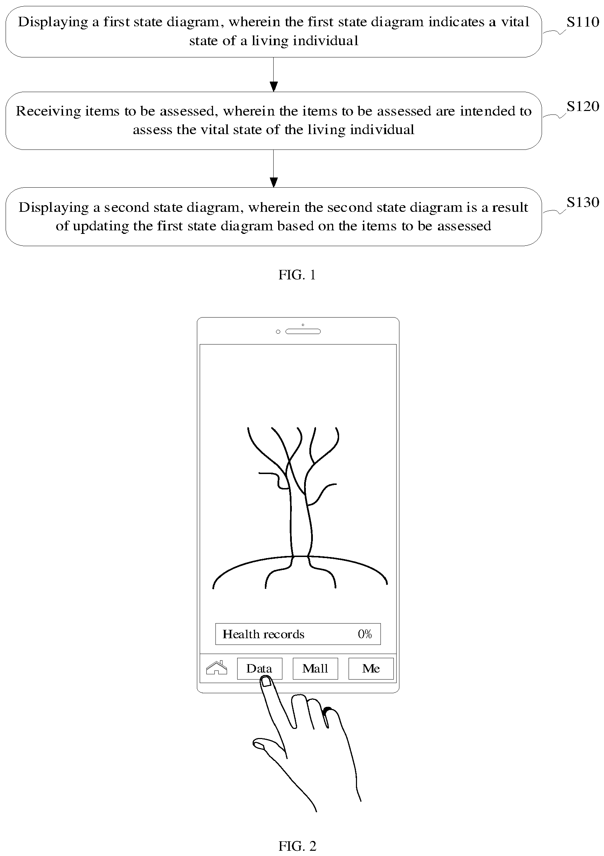

For convenience of understanding and explanation, a method and an apparatus for displaying data, and a terminal and a storage medium thereof according to the embodiments of the present disclosure are described in detail hereinafter with reference to FIGS. 1 to 17.

FIG. 1 is a flowchart of a method for displaying data according to an embodiment of the present disclosure. The method may be performed by a terminal. As shown in FIG. 1, the method includes the steps described hereinafter.

In S110, a first state diagram is displayed, wherein the first state diagram indicates a vital state of a living individual.

In S120, items to be assessed are received, wherein the items to be assessed are intended to assess the vital state of the living individual.

In S130, a second state diagram is displayed, wherein the second state diagram is a result of updating the first state diagram based on the items to be assessed.

In the method for displaying data according to the embodiment of the present disclosure, the first state diagram indicating the vital state of the living individual is displayed, and the first state diagram is updated based on the items to be assessed in response to receiving the items to be assessed to display the second state diagram, such that the state diagram indicating the vital state of the living individual changes with the change of the items to be assessed. Thus, the influence of the items to be assessed on the vital state of the living individual is shown effectively and visually, enabling the user to visually grasp the vital state of the living individual and improving the user experience.

Optionally, the first state diagram and the second state diagram may only be contents displayed in some regions of a terminal operation interface. That is, in case of displaying the first state diagram, the terminal actually displays a first operation interface which includes the first state diagram. In case of displaying the second state diagram, the terminal actually displays the first operation interface which includes the second state diagram. Furthermore, the items to be assessed may be input in the first operation interface or displayed in another operation interface displayed in the terminal. For example, the items to be assessed may be received in a second operation interface displayed in the terminal. The implementation process of the method for displaying data according to the embodiment of the present disclosure is explained hereinafter by taking the embodiment in which the terminal displays the first state diagram and the second state diagram in the first operation interface and receives the items to be assessed in the second operation interface as an example. In case that the terminal displays the first state diagram and the second state diagram and receives the items to be assessed in an actual example, reference is made to the corresponding contents in the following examples for the practice of the method for displaying the data according to the embodiment of the present disclosure.

At this time, the S110 includes: the terminal displays the first operation interface, wherein the first operation interface includes the first state diagram, and the first state diagram indicates the vital state of the living individual.

In response to the user needs to use the terminal to display the vital state of the living individual, the user may operate the terminal to instruct the terminal to display the first operation interface. The first operation interface may be a main interface when the terminal provides a function of displaying the vital state of the living individual. That is, in response to the user needs to use the terminal to provide the function, the terminal displays the first operation interface. The first operation interface includes the state diagram indicating the vital state of the living individual.

For example, as shown in FIG. 2, the terminal of the user is installed with a client providing the function of displaying the vital state of the living individual, and the user can tap a "data" button on a client operation interface by touching to call the first operation interface, such that the terminal displays the first operation interface, and the first operation interface includes the first state diagram.

Optionally, the first state diagram may be a state diagram that the user does not input the items to be assessed of the living individual. At this time, the first state diagram reflects an initialization state of the vital state of the living individual; or, the first state diagram may be a state diagram reflecting the vital state assessed based on the items to be assessed input by the user last time. The items to be assessed input last time interprets the items to be assessed input in a previous process for viewing the state diagram with respect to this viewing.

In another embodiment, the first state diagram is a state diagram showing the vital state of the living individual, and may be, such as, a tree structure diagram; or, the state diagram may be a structure diagram with another shape as long as different shapes of the state diagram can show vital states of the living individual in different time periods. FIG. 2 is a schematic diagram showing that the first state diagram is the tree structure diagram.

In the embodiment of the present disclosure, a biological individual may be the user himself/herself, or may be a person or an animal (such as a pet) that the user cares about. At this time, the vital state of the living individual includes the health state of the living individual. In addition, the method for displaying data according to the embodiment of the present disclosure may also display the vital state of an anthropomorphic biological individual. For example, the method for displaying data according to the embodiment of the present disclosure may also display a growth state of a game character in a game, which is not specifically limited in the embodiments of the present disclosure.

By S120, the terminal receives an operation instruction triggered on the first operation interface, switches the operation interface displayed in the terminal to a second operation interface based on the operation instruction, and receives items to be assessed input on the second operation interface. The operation instruction instructs the terminal to display the second operation interface. The items to be assessed are intended to assess the vital state of the living individual.

In the embodiment of the present disclosure, in response to the user needs to input the items to be assessed, the user may input an operation instruction on the first operation interface to enable the terminal to display the second operation interface, such that the user can input items to be assessed of the user in the second operation interface. As shown in FIG. 2, the user may tap a "health records" icon in FIG. 2 to input the operation instruction on the first operation interface; and at this time, the terminal may display the second operation interface based on the tap operation of the user.

The items to be assessed are the basis for assessing the vital state of the living individual. That is, the terminal or server may assess the vital state of the living individual based on the items to be assessed. Optionally, the items to be assessed may include monitoring information and basic information. The basic information may include at least one of: age, height/weight, marital state, disease history, smoking, drinking, living environment, medication, and the like. The monitoring information may include at least one of: daily exercise steps, daily sleep duration, blood pressure, heart rate, body temperature, blood sugar, uric acid, cholesterol, and the like. Here, the basic information may be manually input by the user, and the monitoring information may be automatically uploaded by a monitoring instrument having a binding relationship with the terminal.

Optionally, the items to be assessed may also include extended information. For example, the function of displaying the vital state of the living individual provided by the terminal may also include a function of answering health knowledge-related questions. At this time, the items to be assessed may also include question answering of the user. For another example, the items to be assessed may also include disease prediction of the living individual, such as the prediction of the living individual suffering from hypertension, diabetes, intermittent claudication, and osteoporosis.

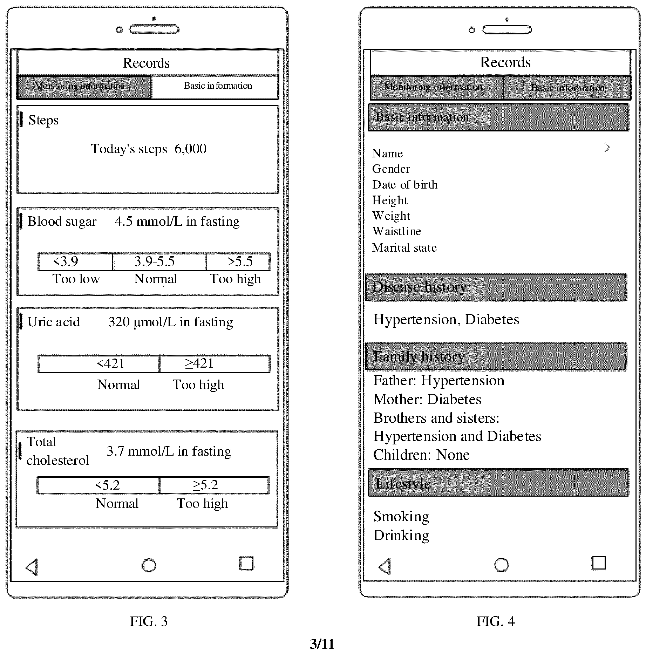

In an exemplary embodiment, as shown in FIGS. 3 and 4, in response to tapping the "health records" icon in FIG. 2 by the user, the second operation interface is displayed in the terminal, and includes two labels. A form of one of the labels allows input of the monitoring information, and a form of the other label allows input of the basic information. As shown in FIG. 3, the monitoring information includes: 6,000 today's exercise steps, 4.5 mmol/L of fasting blood glucose, 320 μmol/L of fasting uric acid, and 3.7 mmol/L of fasting total cholesterol. As shown in FIG. 4, the basic information includes: name, gender, date of birth, height, weight, waistline, marital state, disease history of hypertension and diabetes, family disease history of father suffering from hypertension, mother suffering from diabetes and brothers and sisters suffering from hypertension and diabetes, and lifestyle of smoking and drinking.

By S130, the terminal switches the operation interface displayed in the terminal to the first operation interface based on the items to be assessed, wherein the first operation interface includes a second state diagram which is a result of updating the first state diagram based on the items to be assessed.

In response to completing input of the items to be assessed, the user may further operate the second operation interface, such as taping an OK button, to indicate the terminal that the input of the items to be assessed has been completed, such that the terminal can process the received items to be assessed and display influence of the items to be assessed on the first state diagram in the first operation interface. The influence may be shown by change of the second state diagram relative to the first state diagram. That is, in response to completing the input of the items to be assessed, the terminal may display the second state diagram in the first operation interface based on the items to be assessed, wherein the second state diagram is a result of updating the first state diagram based on the items to be assessed.

In a first example, the terminal may assess the vital state of the living individual based on the items to be assessed, and display the second state diagram based on an assessment result of the vital state of the living individual.

In a second example, the operation of assessing the vital state of the living individual based on the items to be assessed may be performed by the server. At this time, the terminal may send the items to be assessed to the server, such that the server can assess the vital state of the living individual based on the items to be assessed, and the server can send an assessment result of the vital state of the living individual to the terminal. Accordingly, the terminal may receive the assessment result of the vital state of the living individual sent by the server, and switch the operation interface displayed in the terminal to the first operation interface based on the assessment result of the vital state of the living individual.

In the second example, the terminal may encapsulate the received items to be assessed, and send the encapsulated items to be assessed to the server, such that the server can analyze the items to be assessed, and return the analysis result to the terminal. Thus, the terminal can display the second state diagram in the first operation interface.

Optionally, the second state diagram may be a state diagram regenerated by the terminal based on the assessment result; or, the second state diagram may be a state diagram acquired by the terminal dynamically processing the first state diagram based on the assessment result, which is not specifically limited in the embodiments of the present disclosure.

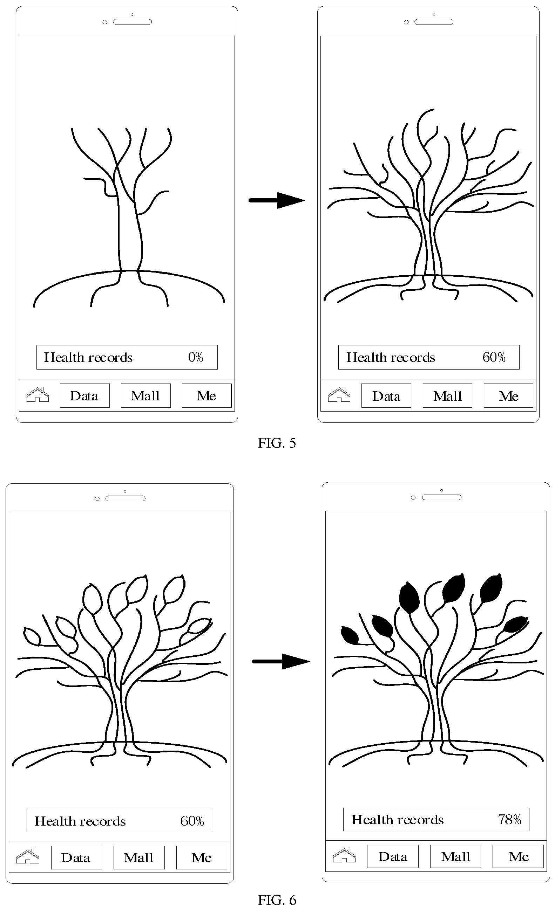

In order to better understand the practice of displaying the second state diagram in the first operation interface by the terminal based on the items to be assessed, the practice of displaying the second state diagram in the first operation interface is explained hereinafter by taking analysis by the server based on the items to be assessed as an example with reference to FIGS. 5 to 13.

Optionally, the example of the second state diagram may at least include the following.

In a first example of the second state diagram, the second state diagram may be a result of growing the first state diagram based on an integrity degree of the items to be assessed. Here, the growing may include positive growing and negative growing of the first state diagram.

Optionally, a total number of items to be assessed that are required to be input may be set. In response to inputting items to be assessed in the second operation interface, a ratio of a total number of the input items to be assessed to the total number of the required items to be assessed may be determined as an integrity degree of the items to be assessed. Furthermore, if the integrity degree of the displayed first state diagram is less than the integrity degree in response to inputting the items to be assessed, the second state diagram is a result of positively growing the first state diagram. If the integrity degree of the displayed first state diagram is greater than the integrity degree in response to inputting the items to be assessed, the second state diagram is a result of negatively growing the first state diagram.

The integrity degree of the items to be assessed input in the second operation interface may be acquired by comparing a total number of input items to be assessed of the displayed first state diagram for triggering the second operation interface and a total number of the items to be assessed input in the second operation interface. For example, in case that a size of the state diagram reflects the integrity degree of the items to be assessed, in response to inputting the items to be assessed in the second operation interface, if the number of the items to be assessed is less than that of the items to be assessed of the displayed first state diagram, it means that an integrity degree of the items to be assessed in response to the items to be assessed in the second operation interface is less than that of the items to be assessed of the displayed first state diagram. Thus, the first state diagram may be negatively processed. That is, the second state diagram may be acquired by reducing the size of the first state diagram. For another example, in case that the size of the state diagram reflects the integrity degree of the items to be assessed, in response to inputting the items to be assessed in the second operation interface, if the number of the items to be assessed is greater than that of the items to be assessed of the displayed first state diagram, it means that the integrity degree of the items to be assessed in response to inputting the items to be assessed in the second operation interface is greater than that of the items to be assessed of the displayed first state diagram. Thus, the first state diagram may be positively processed. That is, the second state diagram may be acquired by increasing the size of the first state diagram.

Furthermore, the total number of the items to be assessed that are required to be input may be set, and different ratios of the total number of the input items to be assessed to the total number of the items to be assessed that are required to be input correspond to state diagrams of different sizes; or, different ratio ranges may be set to correspond to the state diagrams of the different sizes. For example, in case that the ratio is in the range of [0, 15%], the size of the state diagram is set as a first-order size. In case that the ratio is in the range of (15%, 45%], the size of the state diagram is set as a second-order size. In case that the ratio is in the range of (45%, 70%], the size of the state diagram is set as a third-order size. In case that the ratio is in the range of (70%, 90%], the size of the state diagram is set as a fourth-order size. In case that the ratio is in the range of (90%, 100%], the size of the state diagram is set as a fifth-order size.

Optionally, in response to receiving the items to be assessed input in the second operation interface, the terminal may send all the input items to be assessed to the server. In response to receiving the items to be assessed, the server may count the number of the items to be assessed and determine the size of the state diagram based on the number of the items to be assessed, such that the terminal can adjust the first state diagram to acquire the second state diagram based on the determined size. Here, the number of the items to be assessed interprets the total number of the items to be assessed. In addition, different weights may be set for different items to be assessed based on different influence degrees of the items to be assessed on the vital state of the living individual, such that the size of the state diagram can be determined by referring to the weight and the integrity degree of the items to be assessed.

It can be understood that a mapping relationship between the sizes of the state diagrams and the number of the items to be assessed may be stored in the server, and different numbers of the items to be assessed correspond to the different sizes of the state diagrams.

In an embodiment, the mapping relationship may also be interpreted as a corresponding relationship between serial numbers of state diagrams and the number of the items to be assessed. Here, the state diagrams with different serial numbers have different sizes. For example, the server may store a plurality of state trees identified by a plurality of serial numbers respectively. The state trees identified by the different serial numbers have different sizes. In addition, the serial numbers may correspond to number ranges of the items to be assessed. For example, the serial number 1 corresponds to the number range of no more than five items to be assessed; the serial number 2 corresponds to the number range of more than 5 and no more than 10 items to be assessed; the serial number 3 corresponds to the number range of more than 10 and no more than 15 items to be assessed; the serial number 4 corresponds to the number range of more than 15 and no more than 20 items to be assessed; and the serial numbers 1 to 4 respectively identify state trees with different sizes, and the greater the serial number is, the larger the size of the identified state tree is.

In response to the user inputting a plurality of items to be assessed in the second operation interface, the terminal may send the items to be assessed to the server, and the server may count the number of the items to be assessed input in this time, determine a serial number corresponding to the items to be assessed based on the mapping relationship between the serial numbers of the state diagrams and the number range of the items to be assessed, and determine a state tree corresponding to the items to be assessed input this time accordingly. For example, the user inputs 12 items to be assessed, the corresponding state tree with the serial number 3 is determined. An analysis result returned by the server to the terminal carries the serial number 3 of the state tree, or a size of the state tree is directly added to the analysis result based on the serial number 3.

In response to receiving the analysis result sent by the server, the terminal may determine the size of the second state diagram, and perform growth processing on the first state diagram, such as directly replacing the first state diagram with the size indicated by the analysis result to display the second state diagram with the size indicated by the analysis result on the first operation interface, such that the second state diagram is presented as the result of growing the first state diagram.

For example, as shown in FIG. 5, if the state diagram is a tree diagram, the left diagram in FIG. 5 illustrates a first state tree, and the right diagram in FIG. 5 illustrates a second state tree. Based on the serial number in the analysis result, the terminal determines the sizes of all parts of the second state tree to be displayed, and generates the size of the second state tree based on the sizes, wherein the sizes include sizes of a trunk part and a leaf part. Then, the terminal replaces pictures of corresponding parts in the first state tree with pictures of the all parts of the second state tree to be displayed, such that the second state tree displayed in the terminal is presented as a result of increasing a diameter of the trunk and an area of leaves, or even a result of further increasing a density of the leaves (not shown in FIG. 5) based on the first state tree. That is, the second state tree is presented as a result of positively growing the first state tree.

It can be understood that by completing the items to be assessed many times by the user, the process of gradually increasing the state diagram is presented on the first operation interface. That is, the first state diagram may be an initial diagram or a state diagram displayed by the terminal prior to completing the items to be assessed this time. The second state diagram may be the state diagram in response to completing the items to be assessed this time.

In a second example of the second state diagram, the second state diagram is a result of assessing the vital state of the living individual based on the items to be assessed and adjusting a color of the first state diagram based on an assessment result of the vital state of the living individual.

In this example, different colors of the state diagrams may be adopted to reflect different vital states of the living individual. For example, in case that the vital state of the living individual includes a health state of the living individual and the state diagram is a tree structure diagram, the color of leaves in the tree structure diagram may be adopted to represent the health state of the living individual. The greener the leaves in the tree structure diagram are, the healthier the living individual is; and the yellower the leaves in the tree structure diagram are, the unhealthier the living individual is. Accordingly, in response to dynamically processing the first state diagram, the terminal may adjust the color of the first state diagram, such that the second state diagram is presented as a result of adjusting the color of the first state diagram.

In response to receiving the items to be assessed sent by the terminal, the server may analyze each of the items to be assessed, assign values to all the items to be assessed according to a predetermined rule, and assess the vital state of the living individual based on the assigned values of the items to be assessed. Here, the assigned values of the items to be assessed reflect an influence degree of the items to be assessed on the vital state of the living individual, namely, a loss degree to the vital state of the living individual. For example, the assigned value reflecting general influence may be less than 2, the assigned value reflecting moderate influence may be 2-3, and the assigned value reflecting severe influence may be more than 3. That is, if the assigned value of each of the items to be assessed is greater than 2, it means that the items to be assessed are abnormal. For the item under assessment reflecting a sleep duration, if the sleep duration input by the user is 5 hours, a corresponding assigned value is 3, which means that the item under assessment is abnormal; if the sleep duration input by the user is 9 hours, a corresponding assigned value is 1, which means that the item under assessment is normal.

In response to assessing the vital state of the living individual based on the assigned values of the items to be assessed, the server may count the number of each of values in the assigned values of the items to be assessed, and determine the color of the state diagram based on the numbers of the different values. For example, the number of items to be assessed whose assigned values is greater than or equal to 3 may be counted, and the color of the state diagram is determined based on the counted number, such that the vital state of the living individual can be shown by the color.

It can be understood that the server may store a mapping relationship between numbers of abnormal items to be assessed and sizes of the state diagrams, such as a mapping relationship between the number of items to be assessed with an assigned value greater than 2 and a color of the state diagram. For example, in case that the state diagram is a state tree, if the assigned values of the items to be assessed are less than 2, the leaves of the state tree are green, which means that all the items to be assessed are normal; if the assigned values of the items to be assessed include values not less than 2 and not greater than 5, the leaves of the state tree are light yellow, which means that the items to be assessed are slightly abnormal; if the assigned values of the items to be assessed include values greater than 5 and not greater than 10, the leaves of the state tree are yellower, which means that the items to be assessed are seriously abnormal; and so on.

In response to statistically analyzing the assigned values of the items to be assessed input by the user and determining the number of the abnormal items to be assessed, the terminal may determine the color of the state diagram to be displayed, such as the color of the leaves of the state tree. Furthermore, a corresponding relationship between the colors of the state diagrams and the numbers of the items to be assessed may be interpreted as a corresponding relationship between serial numbers of the state diagrams and the numbers of the items to be assessed. The different serial numbers of the state diagrams correspond to the different colors of the state diagrams. Here, the analysis result returned by the server to the terminal may include the serial number of the state diagram, or may include the color of the state diagram.

Accordingly, the terminal may acquire the color to be adjusted by analyzing the analysis result, and adjust the first state diagram displayed by the terminal into a second state diagram based on the color. The second state diagram is acquired by color adjustment based on the first state diagram, for example, replacing a color in the first state diagram with a corresponding color indicated by the analysis result, such that the second state diagram is presented as a result of adjusting the color of the first state diagram. As shown in FIG. 6, the left diagram in FIG. 6 illustrates a first state tree, and the right diagram in FIG. 6 illustrates a second state tree whose leaf color has changed compared with that of the first state tree.

Optionally, the items to be assessed and the corresponding assigned values may be shown in Table 1.

Table 1

| | | Range | Assigned value |
|---|---|---|---|
| Basic information | Age | ≤ 45 | 0 |
| | | (45, 55] | 1 |
| | | (55, 65] | 2 |
| | | (65, 70] | 3 |
| | | (70, 75] | 4 |
| | | > 75 | 5 |
| | Body mass index (BMI) | < 18.5 | 1 |
| | | [18.5,24) | 0 |
| | | [24, 28) | 1 |
| | | [28, 32) | 2 |
| | | > 32 | 3 |
| | Marital state | Single | 0 |
| | | Married | 0 |
| | | Widowed | 2 |
| | | Divorced | 2 |
| | Disease history | Hypertension | 3 |
| | | Diabetes | 3 |
| | | Coronary heart disease | 4 |
| | | Chronic obstructive pulmonary disease | 3 |
| | | Malignant tumor | 4 |
| | | Serious mental disorder | 3 |
| | | Tuberculosis | 3 |
| | | Hepatitis | 3 |
| | | Others | 2 |
| | Smoking | Never | 0 |
| | | Quit | 2 |
| | Drinking | Never | 0 |
| | | Occasional | 1 |

(continued)

| | | | |
|---|---|---|---|
| | | Frequent | 2 |
| | | Everyday | 3 |
| | Living environment | First-class town | 2 |
| | | Second-class town | 1 |
| | | Rural area | 0 |
| | Medication | Antihypertensive drug | 2 |
| | | Hypoglycemic drug | 2 |
| | | Lipid-lowering drug | 2 |
| | | Uric acid-lowering drug | 2 |
| | | Heart diseases drug | 2 |
| | | Asthma relief drug | 2 |
| | | Others | 1 |
| Monitoring information | Steps/day | ≥ 6000 steps | 0 |
| | | [4000, 6000) | 1 |
| | | < 4000 steps | 2 |
| | Sleep duration/day | < 6 hours | 2 |
| | | [6, 7) | 1 |
| | | [7,8) | 0 |
| | | [8,10) | 1 |
| | | > 10 hours | 2 |
| | Blood pressure | Hypotension | 2 |
| | | Normal blood pressure | 0 |
| | | Prehypertension | 0 |
| | | Stage-1 high blood pressure | 2 |
| | | Stage-2 high blood pressure | 3 |
| | | Stage-3 high blood pressure | 4 |
| | Heart rate | [60, 100] | 0 |
| | | < 60, or > 100 | 2 |
| | Body temperature | < 36.2 | 1 |
| | | [36.2, 37.3) | 0 |
| | | [37.3, 38) | 2 |
| | | [38,41] | 3 |
| | | > 41 | 4 |

(continued)

| | | Normal (3.9, 6.1) | 0 |
|---|---|---|---|
| | Fasting blood glucose | Impaired fasting blood glucose (6.1, 7.0) | 1 |
| | | Mild diabetes (7.0, 8.4) | 2 |
| | | (Moderate diabetes (8.4, 11.1)) | 3 |
| | | Severe diabetes (> 11.1) | 4 |
| | Uric acid | Male (149, 416) Female (89, 357) | 0 |
| | | Other ranges | 3 |
| | Total cholesterol | < 2.83 | 2 |
| | | (2.83, 5.20) | 0 |
| | | (5.2, 6.45) | 2 |
| | | > 6.45 | 3 |
| | Interaction knowledge (reflecting the question answering for k times recently, kn being the total number of answers for the k times, and m being the total number of correct answers) | $m \geq kn*0.8$ | 0 |
| | | $kn*0.6 \leq m < kn*0.8$ | 1 |
| | | $m \leq kn*0.6$ | 2 |
| | Hypertension | Low risk | 0 |
| | | Moderate risk | 2 |
| | | High risk | 3 |
| | Diabetes | Risk score < 25 | 0 |
| | | Risk score $\geq$ 25 | 2 |
| Disease risk prediction | Intermittent claudication | Incidence p | 3*p |
| | Osteoporosis | Osteoporosis self-assessment tool | 0 |
| | | for Asians (OSTA) index > -1 | |
| | | -4 $\leq$ OSTA index $\leq$ -1 | 1 |
| | | -4 $\leq$ OSTA index | 2 |

It should be noted that if the user does not input the date of birth or age, the server may calculate an assigned value of the age by using a set default value. If weight and height are not input, an assigned value of the BMI may be calculated by using a default value. If daily steps are not input, a default assigned value 2 may be set as an assigned value of the daily steps. If interaction knowledge is not input, the default assigned value 2 may be set as an assigned value of the interaction knowledge, while default assigned values of other items to be assessed may be 0.

Optionally, in order to comprehensively analyze the assigned values of the items to be assessed, an algorithm may also be provided, and calculates a score of the vital state of the living individual based on the assigned values of the items to be assessed. Accordingly, a third icon may also be displayed on the first operation interface, and interprets a score acquired by assessing the vital state of the living individual based on the assigned values of the items to be assessed.

In an embodiment, the score may be automatically calculated based on the assigned values of the input items to be assessed.

In another embodiment, an assessment icon may be displayed on a display interface of at least one of the first state diagram and the second state diagram. Accordingly, the method further includes: receiving a select operation of the assessment icon; acquiring a score for assessing the vital state of the living individual based on the items to be assessed with instruction of the select operation; and displaying a third icon on the display interface of the at least one state diagram, wherein the third icon interprets the score. That is, in this embodiment, the operation of calculating the score based on the assigned values of the input items to be assessed can be performed with triggering of the user's select operation of the assessment icon.

In addition, the above two operations of calculating the score may be performed by the terminal or the server. In case that each of the operations is performed by the server, the terminal may send the input items to be assessed to the server in response to acquiring the input items to be assessed, such that the server can calculate the score based on the input items to be assessed, and the terminal receives the score calculated by the server.

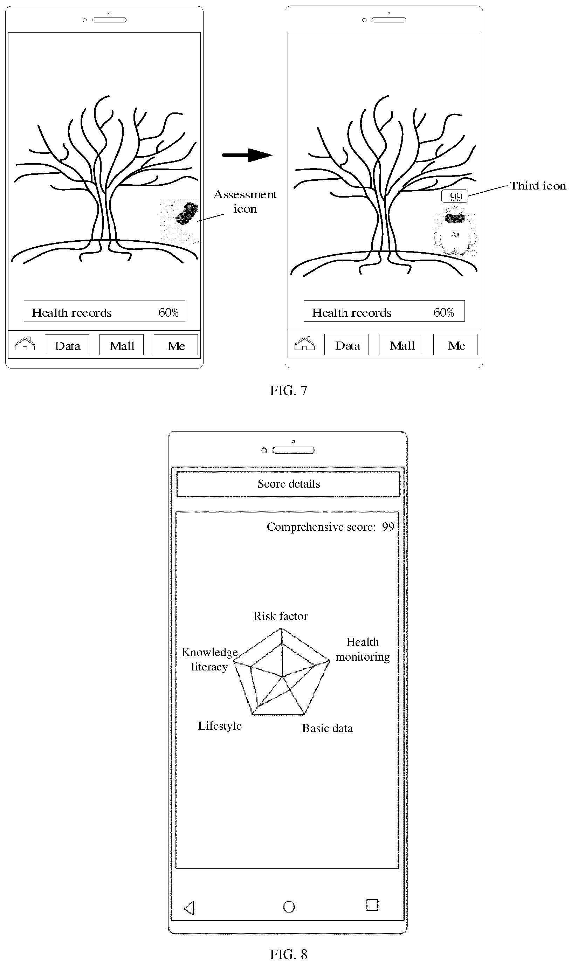

Further, in case that the third icon is displayed on the first operation interface, the method for displaying data may further include that the terminal receives the select operation of the third icon by the user, and displays score details of the score based on the select operation of the third icon. In an exemplary embodiment, as shown in FIG. 7, in response to receiving the select operation of the assessment icon, the terminal acquires the score for assessing the vital state of the living individual based on the items to be assessed, and displays the score using the third icon in FIG. 7. As shown in FIG. 7, the third icon indicates that the score of the vital state of the living individual is 99 points. In response to receiving the select operation of the third icon, the terminal may display the score details as shown in FIG. 8 based on the select operation. The score details indicate the influence of risk factors, knowledge literacy, lifestyle, basic data and health monitoring on the score of the vital state.

Optionally, a process of calculating the score of the vital state of the living individual based on the assigned values of the items to be assessed are described hereinafter.

In S1, a basic score is determined.

In the embodiments of the present disclosure, a default total score of the vital state may be set to 100 points. The basic score may be calculated based on the assigned value of each of the items to be assessed input by the user. In an embodiment, an example of calculating the basic score is described as follows.

In case that the assigned values of all the items to be assessed input by the user are ≤2, it may be determined that the basic score is 100 points. In case that the assigned values of all the items to be assessed input by the user include an assigned value of 3 but do not include an assigned value ≥4, it may be determined that the basic score is 85 points. In case that the assigned values of all the items to be assessed input by the user include the assigned value ≥4, it may be determined that the basic score is 70 points. It should be noted that a condition for calculating the basic score may be adjusted according to actual needs, and the above condition is only an example for the implementation.

In S2, a weight assigned value is calculated.

In S21, a total assigned value is acquired by calculating a sum of assigned values of all items to be assessed.

In S22, an assigned value coefficient is calculated based on the total assigned value.

In an optional embodiment, the total assigned value and the assigned value coefficient satisfy: the assigned value coefficient = 1- total assigned value/100.

In S23, an assigned value constant b is determined based on a distribution interval of the assigned value coefficient.

Optionally, a corresponding relationship between distribution intervals of the assigned value coefficients and assigned value constants may refer to Table 2.

Table 2

| Distribution interval of assigned value coefficient | Assigned value constant |
|---|---|
| [0, 0.1) | 90 |
| [0.1, 0.3) | 70 |
| [0.3, 0.5) | 50 |
| [0.5, 0.7) | 30 |
| [0.7, 1] | 0 |

S24, the weight assigned value is calculated based on the total assigned value, the assigned value coefficient, and the assigned value constant.

In an optional embodiment, the total assigned value, the assigned value coefficient, the assigned value constant, and the weight assigned value may satisfy: the weight assigned value = (the total assigned value - the assigned value constant) $\times$ the assigned value coefficient + the assigned value constant.

In S3, a score of the vital state of the living individual is calculated based the basic score and the weight assigned value.

In an optional embodiment, the basic score, the weight assigned value and the score of the vital state of the living individual satisfy: the score of the vital state of the living individual = the basic score - the weight assigned value.

Optionally, the operation of calculating the score of the vital state of the living individual based on the assigned values of the items to be assessed may take the number of predetermined items to be assessed as a triggering condition. For example, in case that the total number of the items to be assessed input by the user reaches 12, the operation of calculating the score of the vital state of the living individual can be triggered. In case that the total number of the items to be assessed input by the user does not reach 12, the operation of calculating the score of the vital state of the living individual is not performed.

In a third example of the second state diagram, the second state diagram is a result of adding a first icon onto the first state diagram. The first icon indicates an association relationship between the living individual and a specified defect. The association relationship is acquired based on the analysis of the items to be assessed. In an embodiment, in case that the vital state of the living individual includes a health state of the living individual, the association relationship between the living individual and the specified defect may refer to a probability that the living individual has the specified defect.

In an embodiment, an icon for prompting to assess the association relationship between the living individual and the specified defect may be displayed in the first operation interface. The user may tap the icon to instruct the terminal to assess the association relationship between the living individual and the specified defect, and to use the first icon to indicate an assessment result. Here, in response to the icon tapped by the user, the terminal may display a tip for prompting the user to input items to be assessed that are required for assessing the association relationship between the living individual and the specified defect. In response to the input of instructions completed by the user, the terminal may assess based on the items to be assessed input by the user, and use the first icon to indicate the assessment result.

In another embodiment, the process of assessing the association relationship between the living individual and the specified defect based on the items to be assessed may also be automatically performed. For example, in response to detecting the acquired items to be assessed required for assessing the association relationship between the living individual and the specified defect, the terminal may automatically assess the association relationship between the living individual and the specified defect based on the acquired items to be assessed, and use the first icon to indicate the assessment result.

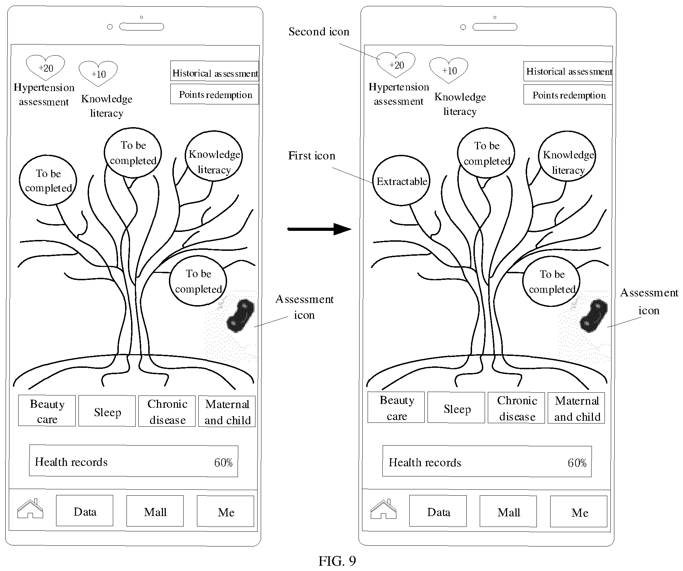

As shown in FIG. 9, a "to be completed" icon may be displayed in the terminal, and prompts to assess the association relationship between the living individual and the specified defect. In response to the icon tapped by the user, the terminal may assess the association relationship between the living individual and the specified defect based on the items to be assessed, and use an "extractable" first icon to indicate the assessment result, so as to prompt the user to view the association relationship between the living individual and the specified defect.

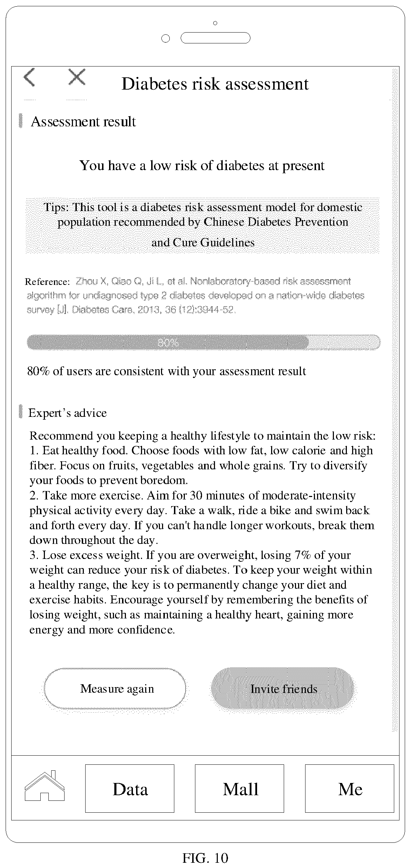

Corresponding to the third example, the method for displaying data may further include: receiving a select operation of the first icon. Based on the select operation of the first icon, analysis of the association relationship between the living individual and the specified defect is displayed. For example, in response to the user tapping a prompt message for prompting to view the association relationship between the living individual and diabetes, the terminal may display a diabetes assessment result of the living individual as shown in FIG. 10.

Optionally, in order to present a more complete vital state of the living individual, the server may also assess an association relationship between the living individual to which the items to be assessed belong and one or more specified defects based on a predetermined algorithm and the received items to be assessed. The specified defects may include osteoporosis, hypertension, intermittent claudication, diabetes, and the like. For example, based on the predetermined algorithm and the input items to be assessed, the server may analyze and assess whether the user has risks such as hypertension, intermittent claudication and diabetes, and levels of the risks. Accordingly, the server may return an analysis result including an assessment result to the terminal, such that the terminal can dynamically process the first state diagram based on the assessment result. That is, one or more first icons corresponding to the specified defects are added onto the first state diagram. Here, the first icon corresponding to the specified defect indicates the association relationship between the user and the specified defect.

For example, in response to the server assessing the association relationship between the living individual and diabetes, intermittent claudication and diabetes, and returning an analysis result to the terminal, the terminal may add one first icon corresponding to diabetes, one first icon corresponding to intermittent claudication and one first icon corresponding to hypertension onto the first state diagram. The first icon corresponding to diabetes indicates a probability of the living individual suffering from diabetes; the first icon corresponding to intermittent claudication indicates a probability of the living individual suffering from intermittent claudication; and the first icon corresponding to hypertension indicates a probability of the living individual suffering from hypertension.

Optionally, the following formula may be adopted as an assessment algorithm to assess whether the living individual suffering from hypertension. A calculation result of this formula indicates a value of the risk that the user suffers from hypertension in a specified time period in the future (such as in the next four years). In addition, the items to be assessed involved in assessing whether the living individual suffering from hypertension include: gender, age, smoking habit in life, BMI, systolic blood pressure, diastolic blood pressure and family history of whether parents suffering from hypertension.

$$1 - \exp\left[ -\exp\left( \dfrac{\text{Ln}(4) - \left[ 22.94954 + \sum_i x_i \times \beta_i \right]}{0.87692} \right) \right]$$

Here, $x_i$ is an assigned value of an $i^{th}$ item under assessment, and $\beta_i$ is a regression coefficient corresponding to the item under assessment. In addition, in case that the item under assessment is gender, an assigned value of female is 1 and an assigned value of male is 0. Assigned values of the family history whether parents suffering from hypertension are: in case that one parent suffers from hypertension, an assigned value of x is 1, and in case that both parents suffer from hypertension, an assigned value of x is 2. Here, the items to be assessed and the values of corresponding regression coefficient $\beta$ may be determined with reference to Table 3 below.

It should be noted that considering occurrence characteristics of hypertension, in order to ensure the accuracy of estimation, the items to be assessed input by the user may be judged to determine whether the items to be assessed meet an estimation condition, and estimation is performed only when the items to be assessed meet the estimation condition. Here, the items to be assessed involved in the estimation condition include height, weight, age, systolic blood pressure, diastolic blood pressure, and the like.

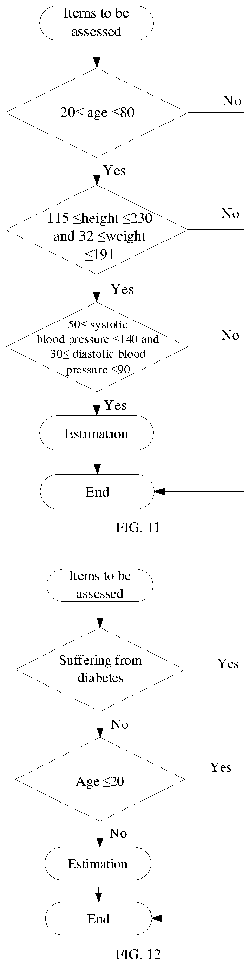

In an embodiment of judging whether the items to be assessed meet the estimation condition, as shown in FIG. 11, in case that the items to be assessed do not meet at least one of an age range, a height range, a weight range, a diastolic blood pressure range and a systolic blood pressure range, the algorithm is directly ended, and the returned analysis result does not include any information about assessment of hypertension. In case that the items to be assessed all meet the age range, the height range, the weight range, the diastolic blood pressure range and the systolic blood pressure range, a probability of the living individual suffering from hypertension is estimated. In addition, in order to improve the user experience, if it is determined that the items to be assessed do not meet at least one of the age range, the height range, the weight range, the diastolic blood pressure range and the systolic blood pressure range, a prompt message of "the estimation is not for you" may be displayed. Here, the age range may be [20 years old, 80 years old], the height range may be [1.15 meters, 2.3 meters], the weight range may be [32 kilograms, 191 kilograms], the diastolic blood pressure range may be [50 mmHg, 140 mmHg], and the systolic blood pressure range may be [30 mmHg, 90 mmHg].

Table 3

| Item under assessment | Regression coefficient |
|---|---|
| Age | -0.15641 |
| Gender | -0.20293 |
| Systolic blood pressure | -0.05933 |
| Diastolic blood pressure | -0.12847 |
| Smoking | -0.19073 |
| Whether parents suffering from hypertension | -0.16612 |
| BMI | -0.03388 |
| Age $\times$ diastolic blood pressure | 0.00162 |

For the assessment of diabetes, the score of each of the items to be assessed of the living individual may be acquired based on the items to be assessed of the living individual, a sum of the scores of all the items to be assessed of the living individual is determined as an assessment score of the user, and a risk of the living individual suffering from diabetes within a specified time period in the future is determined based on the assessment score. For example, if the assessment score is $\geq 25$, it means that the living individual has the risk of diabetes, and the first icon on the first operation interface shown in FIG. 9 may be used to prompt the living individual of the risk of diabetes. For example, "You are at risk of diabetes. Recommend you to screen it in the near future and adopt a healthy lifestyle" may be displayed on the first icon. If the assessment score is < 25, it means that the living individual has no risk of diabetes, and the first icon on the first operation interface shown in FIG. 9 may be used to prompt that the living individual has no risk of diabetes. For example, "You have a low risk of diabetes at present. Recommend you keeping a healthy lifestyle to maintain the low risk" may be displayed on the first icon. Here, in case of assessing whether the living individual suffering from diabetes, the items to be assessed need to be designed include age, systolic blood pressure, waistline, BMI, diabetes history, and gender.

For the risk assessment of diabetes, please refer to Table 4 for a corresponding relationship between the items to be assessed and the scores.

It should be noted that considering occurrence characteristics of diabetes, in order to ensure the accuracy of estimation, the items to be assessed input by the user may be judged to determine whether the items to be assessed meet an estimation condition, and estimation is performed only when the items to be assessed meet the estimation condition. Here, the items to be assessed involved in the estimation condition include: whether the living individual suffering from diabetes, age, and the like.

In an embodiment of judging whether the items to be assessed meet the estimation condition, as shown in FIG. 12, in case that the items to be assessed do not meet an age range and/or the living individual suffers from diabetes, the algorithm is directly ended, and the returned analysis result does not include any information about assessment of diabetes. In case that the items to be assessed meet the age range and the living individual does not suffer from diabetes, a probability of the living individual suffering from diabetes is estimated. In addition, in order to improve the user experience, a prompt message "Please follow the doctor's advice to manage diabetes" may be displayed in case that it is determined that the living individual suffers from diabetes, and a prompt message of "the estimation is not for you" may be displayed in case that the age condition is not met. Here, the age range may be more than 20 years old.

Table 4

| Age (years) | Score | Waistline | Score |
|---|---|---|---|
| 20-24 | 0 | < 75 (male) or < 70 (female) | 0 |
| 25-34 | 4 | 75-79.9 (male) or 70-74.9 (female) | 3 |
| 35-39 | 8 | 80-84.9 (male) or 75-79.9 (female) | 5 |
| 40-44 | 11 | 85-89.9 (male) or 80-84.9 (female) | 7 |
| 45-49 | 12 | 90-94.9 (male) or 85-89.9 (female) | 8 |
| 50-54 | 13 | $\geq$ 95 (male) or $\geq$ 90 (female) | 10 |
| 55-59 | 15 | BMI (kg/m$^2$) | |
| 60-64 | 16 | < 22 | 0 |
| 65-74 | 18 | 22-23.9 | 1 |
| Systolic blood pressure (mmHg) | | 24-29.9 | 3 |
| < 110 | 0 | $\geq$ 30 | 5 |
| 110-119 | 1 | Family history of diabetes | |
| 120-129 | 3 | No | 0 |
| 130-139 | 6 | Yes | 6 |
| 140-149 | 7 | Gender | |
| 150-159 | 8 | Female | 0 |
| $\geq$ 160 | 10 | Male | 2 |

For the assessment of intermittent claudication, the score of each of the items to be assessed of the living individual may be acquired, a sum of the scores of all the items to be assessed of the living individual is determined as an assessment score of the living individual, and a probability of the living individual suffering from intermittent claudication within a specified time period in the future is determined based on the assessment score. Here, the items to be assessed and their scores may refer to Table 5. The assessment score of the living individual and the probability of the living individual suffering from intermittent claudication in the next four years may refer to Table 6.

Table 5

| Item under assessment | Score of item under assessment | | | | | |
|---|---|---|---|---|---|---|
| | 0 | +1 | +2 | +3 | +4 | +5 |
| Age | 45-49 | 50-54 | 55-59 | 60-64 | 65-69 | 70-74 |
| Gender | Female | | | Male | | |
| Serum cholesterol (mg/dL) | < 170 | 170-209 | 210-249 | 250-289 | > 289 | |
| Hypertension | Normal | Prehypertension | Stage-1 high blood pressure | | Stage $\geq$ 2 high blood pressure, and coronary heart disease | |
| Smoking (pcs/day) | 0 | 1-5 | 6-10 | 11-20 | > 20 | |
| Diabetes | No | | | | | Yes |
| Coronary heart disease | No | | | | | Yes |

It should be noted that considering occurrence characteristics of intermittent claudication, in order to ensure the accuracy of estimation, the items to be assessed input by the user may be judged to determine whether the items to be assessed meet an estimation condition, and estimation is performed only when the items to be assessed meet the estimation condition. Here, the items to be assessed involved in the estimation condition include: age, and the like.

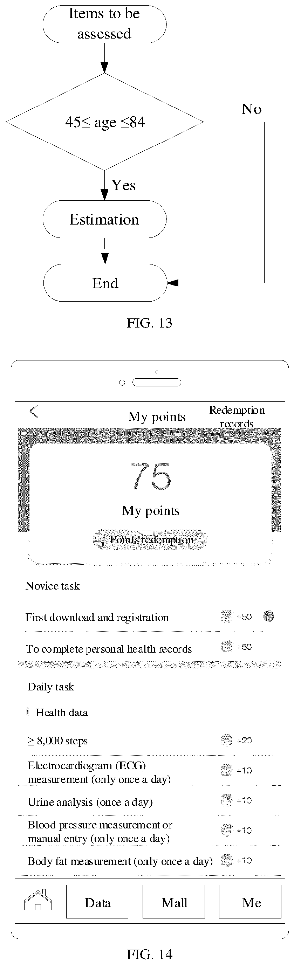

In an embodiment of judging whether the items to be assessed meet the estimation condition, as shown in FIG. 13, in case that the item under assessment does not meet an age range, the algorithm is directly ended, and the returned analysis result does not include any information about assessment of intermittent claudication. In case that the item under assessment meets the age range, a probability of the living individual suffering from intermittent claudication is estimated. In addition, in order to improve the user experience, if it is determined that the item under assessment does not meet the age range, a prompt message of "the estimation is not for you" may be displayed. Here, the age range may be [45 years old, 84 years old].

Table 6

| Assessment score | Probability of living individual suffering from intermittent claudication in the next four years |
|---|---|
| < 10 | Less than 1% |
| 10-12 | 1% |
| 13-15 | 2% |
| 16-17 | 3% |
| 18 | 4% |
| 19 | 5% |
| 20 | 6% |
| 21 | 7% |
| 22 | 8% |
| 23 | 10% |
| 24 | 11% |
| 25 | 13% |
| 26 | 16% |
| 27 | 18% |

(continued)

| Assessment score | Probability of living individual suffering from intermittent claudication in the next four years |
|---|---|
| 28 | 21% |
| 29 | 24% |
| 30 | 28% |

It can be understood that in the embodiments of the present disclosure, other defect indicators, such as osteoporosis, may also be predicted. In an embodiment, an osteoporosis self-assessment tool for Asians (OSTA) may be used for prediction.

In an embodiment, a relationship between an OSTA index of a living individual and a weight and age of the living individual satisfies: OSTA index = (weight-age) $\times 0.2$.

Please refer to Table 7 for a corresponding relationship between the OSTA index of the living individual and a risk level of osteoporosis of the living individual. For example, in case that the OSTA index of the living individual belongs to the range of $(-1,+\infty)$, it can be determined that the risk level of osteoporosis of the living individual is low. In case that the OSTA index belongs to the range of $[-3, -1]$, it can be determined that the risk level of osteoporosis of the living individual is medium. In case that the OSTA index of the living individual belongs to the range of $[-4, -3]$, it can be determined that the risk level of osteoporosis of the living individual is slightly high. In case that the OSTA index of the living individual belongs to the range of $(-\infty, -4]$, it can be determined that the risk level of osteoporosis of the living individual is high.

Table 7

| Index interval | Risk level |
|---|---|
| OSTA index > -1 | Low |
| $-3 \leq$ OSTA index $\leq$ -1 | Medium |
| $-4 \leq$ OSTA index < -3 | Slightly high |
| OSTA index $\leq$ -4 | High |

It should be noted that considering occurrence characteristics of osteoporosis, in order to ensure the accuracy of estimation, the items to be assessed input by the user may be judged to determine whether the items to be assessed meet an estimation condition, and estimation is performed only when the items to be assessed meet the estimation condition. Here, the items to be assessed involved in the estimation condition include: weight, age, and the like.

In an embodiment of judging whether the items to be assessed meet the estimation condition, in case that the items to be assessed do not meet at least one of an age range and a weight range, the algorithm is directly ended, and the returned analysis result does not include any information about assessment of osteoporosis. In case that the items to be assessed meet both the age range and the weight range, a probability of the living individual suffering from osteoporosis is estimated. In addition, in order to improve the user experience, if it is determined that the items to be assessed do not meet at least one of the age range and the weight range, a prompt message of "the estimation is not for you" may be displayed. Here, the weight range may be [25 kg, 125 kg]. The age range may be [35 years old, 70 years old].

It should also be noted that when estimating the association relationship between the living individual and the specified defect, an influence weight of the items to be assessed on the specified defect may also be set based on an influence degree of the items to be assessed on the specified defect, and the association relationship between the living individual and the specified defect may be estimated with reference to the influence weight.

In a fourth example of the second state diagram, the second state diagram is a result of adding a second icon onto the first state diagram. The second icon indicates a processing assessment value of an operation performed by the user. The processing assessment value rewards a specified operation completed by the user to motivate the user. For example, in response to the items to be assessed completed by the user, as shown in FIG. 9, the terminal may display a second icon in the fashion of a floating bubble while displaying the first operation interface to reward the user with points; or, in response to the items to be assessed updated by the user, the terminal may display the second icon in the fashion of the floating bubble while displaying the first operation interface to reward the user with points; or, in response to the user viewing an association relationship between the living individual and hypertension, as shown in FIG. 9, the terminal may display the second icon in the fashion of the floating bubble while displaying the first operation interface to reward the user with points; or, in response to the user viewing interaction knowledge related to the items to be assessed, the terminal may display the second icon in the fashion of the floating bubble while displaying the first operation interface to reward the user with points.

As an example of motivating the user by using the processing assessment value, a redemption icon is displayed in the second state diagram. The method for displaying the data according to the embodiments of the present disclosure further includes: receiving a select operation of the redemption icon; displaying a redemption page of articles to be redeemed by using the processing assessment value based on the select operation of the redemption icon; receiving a select operation on the redemption page; and performing an operation of redeeming an article indicated by the select operation on the redemption page by using the processing assessment value of the user, based on the select operation on the redemption page. Here, the article may be a virtual article such as a discount coupon, or a physical article such as a sleep monitor, a blood glucose monitor, or other health-care devices.

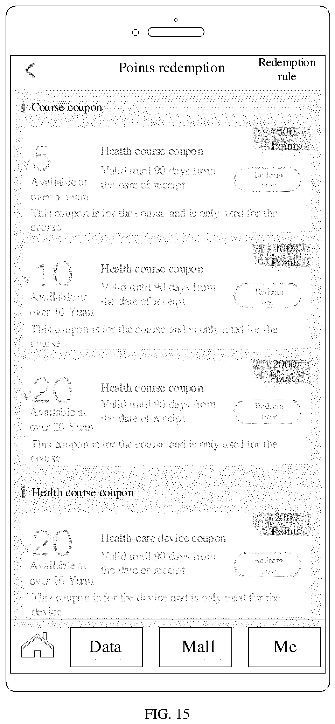

For example, a redemption icon of "points redemption" is displayed in the first operation interface as shown in FIG. 9. In response to the user desiring to redeem an article with points, the user may perform a select operation on the redemption icon. In response to receiving the select operation, the terminal may display an interface for explaining points as shown in FIG. 14, and the current points of the user and a "points redemption" button are displayed in the interface. In response to the user performing a select operation on the "points redemption" button, the terminal displays the redemption page of the articles to be redeemed by using points as shown in FIG. 15. In response to determining the article to be redeemed with points, the user can perform a select operation on the corresponding article. Based on the select operation, the terminal may perform the operation of using the user's points to redeem the article indicated by the select operation on the redemption page, realizing the task of redeeming the article with the points.

It should be noted that the more items to be assessed input by the user are, the more accurate the assessment result of the vital state of the living individual based on the items to be assessed is. Thus, an icon for prompting to complete the items to be assessed is further displayed on a display interface of at least one of the first state diagram and the second state diagram. For example, as shown in FIG. 5, an icon of "health records" may be displayed on each of the first operation interface and the second operation interface, and prompts to complete the items to be assessed. Furthermore, a percentage indicating the integrity degree of the items to be assessed is also displayed on the icon of the "health records," such that the user can know the integrity degree of the input items to be assessed. In addition, encouraging words "completing the health file brings you large points" may be displayed on the "health records" icon to encourage the users to complete the items to be assessed.

In addition, in case that the items to be assessed are uncompleted, an icon for prompting the user to complete the items to be assessed may also be displayed in the first operation interface. For another example, in response to the user viewing the association relationship between the living individual and the specified defect, an icon for prompting the user to update a corresponding item under assessment may also be displayed in the first operation interface, such that re-assessment based on the updated items to be assessed is facilitated.

Optionally, in order to further improve the user experience, the terminal may also store interaction knowledge for helping the user acquire the items to be assessed. An icon (such as a "knowledge literacy" icon in FIG. 9) for prompting the user to view the interaction knowledge related to the items to be assessed may also be displayed on the display interface of at least one of the first state diagram and the second state diagram, so as to prompt the user to view the interaction knowledge related to the items to be assessed by tapping the icon. In addition, the interaction knowledge may be displayed on the operation interface of the terminal in the fashion of a question and answer to stimulate reading interest of the user.

It should be noted that the operation of predicting the vital state in the embodiments of the present disclosure may be performed by using artificial intelligence (AI) technologies. In addition, the method for displaying data according to the embodiments of the present disclosure may be performed by an application installed in the terminal, and the application also has other functions which can share data with functions provided by the method for displaying data according to the embodiments of the present disclosure. For example, the application also has functions of providing beauty care advice, sleep advice, maternal and child advice, chronic disease advice. Moreover, these functions can share data with the functions provided by the method for displaying data according to the embodiments of the present disclosure. Therefore, as shown in FIG. 9, roots of the tree structure shown in FIG. 9 are also linked with icons of "beauty care," "sleep," "maternal and child," and "chronic disease," which indicates that the functions provided by the method for displaying data according to the embodiments of the present disclosure can share data with the functions of "beauty care," "sleep," "maternal and child," and "chronic disease."

In the method for displaying data according the embodiments of the present disclosure, the first state diagram indicating the vital state of the living individual is displayed on the first operation interface, and the first state diagram is updated based on the items to be assessed in response to receiving the items to be assessed; the updated second state diagram is displayed on the operation interface, such that the state diagram indicating the vital state of the living individual changes with the change of the items to be assessed. Thus, the influence of the items to be assessed on the vital state of the living individual is shown effectively and visually, enabling the user to visually grasp the vital state of the living individual and improving the user experience.

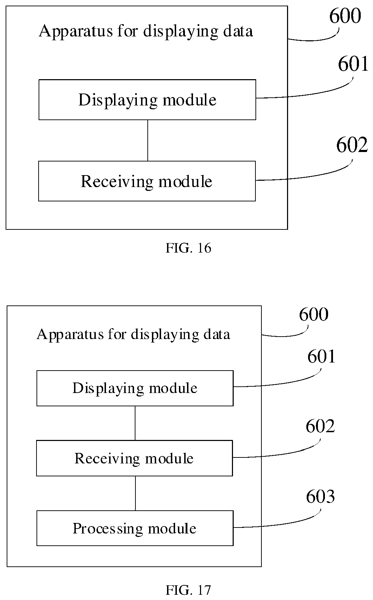

An apparatus for displaying data is further provided according to the embodiment of the present disclosure. As shown in FIG. 16, the apparatus 600 for displaying data includes a display module 601 and a receiving module 602.

The displaying module 601 is configured to display a first state diagram, wherein the first state diagram indicates a vital state of a living individual.

The receiving module 602 is configured to receive items to be assessed, wherein the items to be assessed are intended to assess the vital state of the living individual.

The displaying module 601 is further configured to display a second state diagram, wherein the second state diagram is a result of updating the first state diagram based on the items to be assessed.

In summary, in the apparatus for displaying data according the embodiments of the present disclosure, the displaying module displays the first state diagram indicating the vital state of the living individual on the first operation interface, and updates the first state diagram based on the items to be assessed in response to the receiving module receiving the items to be assessed to display the updated second state diagram on the operation interface, such that the state diagram indicating the vital state of the living individual changes with the change of the items to be assessed. Thus, the influence of the items to be assessed on the vital state of the living individual is shown effectively and visually, enabling the user to visually grasp the vital state of the living individual and improving the user experience.

Optionally, the second state diagram is a result of growing the first state diagram based on an integrity degree of the items to be assessed.

Optionally, the second state diagram is a result of assessing the vital state of the living individual based on the items to be assessed and adjusting a color of the first state diagram based on an assessment result of the vital state of the living individual.

Optionally, the second state diagram is a result of adding a first icon onto the first state diagram, wherein the first icon indicates an association relationship between the living individual and a specified defect, and the association relationship is acquired based on analysis of the items to be assessed.

Optionally, the receiving module 602 is further configured to receive a select operation of the first icon.

Accordingly, the displaying module 601 is further configured to display analysis of the association relationship between the living individual and the specified defect based on the select operation of the first icon.

Optionally, the second state diagram is a result of adding a second icon onto the first state diagram, wherein the second icon indicates a processing assessment value of an operation performed by a user.

Optionally, a redemption icon is displayed in the second state diagram.

The receiving module 602 is further configured to receive a select operation of the redemption icon.

The displaying module 601 is further configured to display a redemption page of articles to be redeemed by using the processing assessment value based on the select operation of the redemption icon.

The receiving module 602 is further configured to receive a select operation on the redemption page.

As shown in FIG. 17, the apparatus 600 for displaying data further includes a processing module 603, configured to perform an operation of redeeming an article indicated by the select operation on the redemption page by using the processing assessment value of the user, based on the select operation on the redemption page.

Optionally, an icon for prompting to complete the items to be assessed is further displayed on a display interface of at least one of the first state diagram and the second state diagram.

Optionally, an icon for prompting to view interaction knowledge related to the items to be assessed is further displayed on a display interface of at least one of the first state diagram and the second state diagram.

Optionally, an assessment icon is displayed on a display interface of at least one of the first state diagram and the second state diagram, and the receiving module 602 is further configured to receive a select operation of the assessment icon.

The processing module 603 is further configured to acquire a score for assessing the vital state of the living individual based on the items to be assessed with instruction of the select operation.

The displaying module 601 is further configured to display a third icon on a display interface of the at least one state diagram, wherein the third icon interprets the score.

Optionally, the receiving module 602 is further configured to receive a select operation of the score.

Accordingly, the displaying module 601 is further configured to display score details of the score based on the select operation of the score.

Optionally, the processing module 603 is further configured to assess the vital state of the living individual based on the items to be assessed.

Accordingly, the displaying module 601 is specifically configured to display the second state diagram based on the assessment result of the vital state of the living individual.

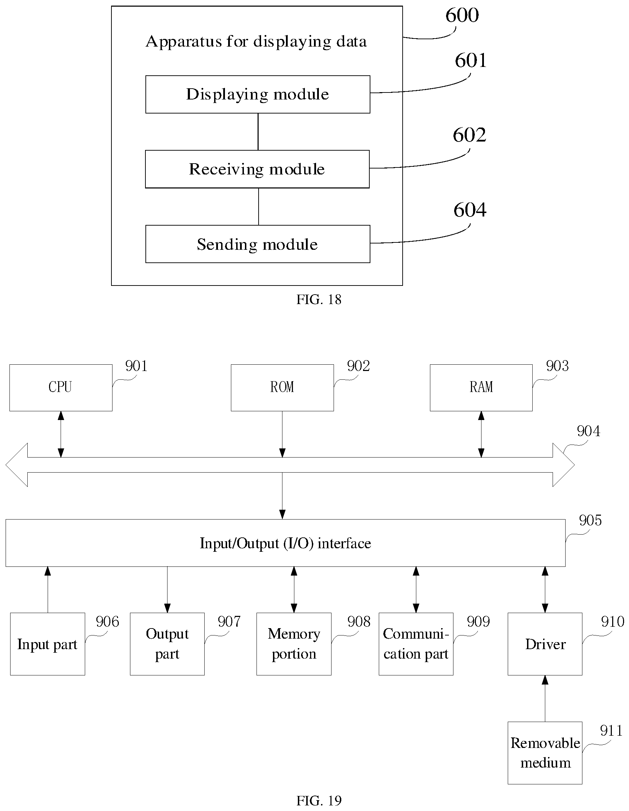

Or, as shown in FIG. 18, the apparatus 600 for displaying data further includes a sending module 604, configured to send the items to be assessed to a server.

Accordingly, the receiving module 602 is further configured to receive an assessment result of the vital state of the living individual sent by the server, wherein the assessment result is acquired based on the items to be assessed.

Accordingly, the displaying module 601 is specifically configured to display the second state diagram based on the assessment result of the vital state of the living individual.

Optionally, both the first state diagram and the second state diagram are tree structure diagrams.

Optionally, the vital state of the living individual includes a health state of the user.

In summary, in the apparatus for displaying data according the embodiments of the present disclosure, the displaying module displays the first state diagram indicating the vital state of the living individual on the first operation interface, and updates the first state diagram based on the items to be assessed in response to the receiving module receiving the items to be assessed to display the updated second state diagram on the operation interface, such that the state diagram indicating the vital state of the living individual changes with the change of the items to be assessed. Thus, the influence of the items to be assessed on the vital state of the living individual is shown effectively and visually, enabling the user to visually grasp the vital state of the living individual and improving the user experience.

A terminal is further provided according to the embodiments of the present disclosure. The device includes a memory, a processor, and a computer program stored on the memory and capable of running on the processor, wherein the program, when run by the processor, causes the processor to perform the method for displaying data according to the embodiments of the present disclosure.

FIG. 19 shows a schematic structure diagram of a terminal of a device, which is suitable for performing the embodiments of the present disclosure. As shown in FIG. 19, the terminal includes a central processing unit (CPU) 901 which may perform various appropriate actions and processing according to a program stored in a read-only memory (ROM) 902 or a program loaded to a random access memory (RAM) 903 from a memory portion 903. Various programs and data required during operation of a system 900 are also stored in the RAM 903. The CPU 901, the ROM 902, and the RAM 903 are connected to one another via a bus 904. An input/output (I/O) interface 905 is also connected to the bus 904.

Components connected to the I/O interface 905 include: an input part 906 including a keyboard, a mouse and the like, an output part 706 including such as a cathode ray tube (CRT), a liquid crystal display (LCD) and a speaker, a storing part 908 including hardware and the like, and a communication part 909 including network interface cards such as an LAN card, a modem, and the like. The communication part 909 performs communication processing via a network such as the Internet. A driver 910 is connected to the I/O interface 905 according to the needs. A removable medium 911 such as a magnetic disk, an optical disk, a magneto-optical disk, or a semiconductor memory is installed on the driver 910 as required so as to enable a computer program read out from the removable medium to be installed into the memory portion 908 according to the actual needs.

In the method for displaying data according to the embodiments of the present disclosure, the process described above with reference to FIG. 1 may be performed as a computer software program. For example, a computer program product including a computer program is further provided according to the embodiments of the present disclosure, wherein the computer program is tangibly contained in a machine-readable medium, and the computer program includes a program code for performing the method as shown in FIG. 1. The computer program may be downloaded and installed from the network via the communication part 909, and/or may be installed from the removable medium 911.

It should be noted that the machine-readable medium provided by the embodiments of the present disclosure may be a computer-readable signal medium or a computer-readable storage medium or any combination of the above two. The computer-readable storage medium can be, for example, but not limited to, an electrical, magnetic, optical, electromagnetic, infrared, or semiconductor system, apparatus, or device, or any combination of the above. More specific examples of the computer-readable storage medium may include, but are not limited to, an electrical connection with one or more wires, a portable computer disk, a hard disk, a RAM, a ROM, an erasable programmable read-only memory (EPROM or flash memory), an optical fiber, a portable compact disk read-only memory (CD-ROM), an optical storage device, a magnetic storage device, or any combination thereof. In an embodiment of the present disclosure, the computer-readable storage medium can be any tangible medium containing or storing a program, which can be used by or in combination with an instruction execution system, apparatus, or device.

In the embodiments of the present disclosure, the computer-readable signal medium may include a data signal propagated in baseband or as a part of a carrier wave, in which the computer-readable program code is carried. This propagated data signal can take various forms, including but not limited to electromagnetic signals, optical signals, or any suitable combination of the above. The computer-readable signal medium may also be any computer-readable storage medium other than the computer-readable storage medium, which can send, propagate or transmit the program for use by or in combination with the instruction execution system, apparatus, or device. The program code stored on the computer readable storage medium can be transmitted by any suitable medium, including but not limited to wireless, electric wire, optical cable, radio frequency (RF), and the like, or any combination thereof.

The flowcharts and block diagrams in the accompanying drawings show the architectures, functions and operations that may be performed by the method for displaying data and computer program products according to the embodiments of the present disclosure. Each block in the flowcharts and block diagrams may interpret one module, one program segment or a part of a code, which includes one or more executable instructions for performing determined logical functions. It should also be noted that in some alternative examples, the function described in one block may be realized in a sequence difference from that described in the accompanying drawings. For example, two consecutive blocks may be performed substantially in parallel, and may be performed in an inverse sequence, depending on the related functions. It should be also noted that each block or a combination of the blocks in the flowcharts and/or block diagrams may be performed by specific software systems for performing determined functions or operations, or by a combination of specific hardware and computer instructions.

The units or modules described in the embodiments of the present disclosure may be performed by software or hardware. The described units or modules may also be arranged in a processor. For example, it can be described as: a processor including a display module and a receiving module. The names of these units or modules do not define the units or modules themselves in some cases. For example, the display module may also be described as "for displaying a second state diagram based on the items to be assessed, wherein the second state diagram is a result of updating the first state diagram based on the items to be assessed."

A computer-readable storage medium is further provided according to the embodiments of the present disclosure. The computer-readable storage medium may be the computer-readable storage medium included in the above apparatus according to the above embodiments, or may be a computer-readable storage medium that exists alone and is not assembled in the device. The computer-readable storage medium stores one or more programs. The one or more programs are run by one or more processors to perform the method for displaying data according to the embodiments of the present disclosure. For example, the method includes: displaying a first state diagram, wherein the first state diagram indicates a vital state of a living individual; receiving items to be assessed, wherein the items to be assessed are intended to assess the vital state of the living individual; and displaying a second state diagram, wherein the second state diagram is a result of updating the first state diagram based on the items to be assessed.

In the embodiments of the present disclosure, the terms "first" and "second" merely describe but not denote or imply any relative importance. The term "a plurality of" means two or more, unless otherwise expressly provided.

The above embodiments of the present disclosure are only examples for clearly explaining the present disclosure, and are not the limitations of the embodiments of the present disclosure. Those skilled in the art should understand that the inventive scope involved in the present disclosure is not limited to the technical solutions formed by specific combinations of the above technical features, but also covers other technical solutions formed by any combination of the above technical features or their equivalent features without departing from the inventive concept. For example, the above technical solutions refer to technical solutions formed by replacing the above features with the technical features with similar functions disclosed in the present disclosure (but not limited to). Here, it is impossible to enumerate all the embodiments. Any obvious changes or variations derived from the technical solutions of the present disclosure are still within the protection scope of the present disclosure.

**Claims**

1.   A method for displaying data, applicable to a terminal, the method comprising:

   displaying a first state diagram, wherein the first state diagram indicates a vital state of a living individual;
   receiving items to be assessed, wherein the items to be assessed are intended to assess the vital state of the living individual; and
   displaying a second state diagram, wherein the second state diagram is a result of updating the first state diagram based on the items to be assessed.

2.   The method for displaying data according to claim 1, wherein the second state diagram is a result of growing the first state diagram based on an integrity degree of the items to be assessed.

3.   The method for displaying data according to claim 1 or 2, wherein the second state diagram is a result of assessing the vital state of the living individual based on the items to be assessed and adjusting a color of the first state diagram based on an assessment result of the vital state of the living individual.

4.   The method for displaying data according to any one of claims 1 to 3, wherein the second state diagram is a result of adding a first icon onto the first state diagram, the first icon indicates an association relationship between the living individual and a specified defect, and the association relationship is acquired based on analysis of the items to be assessed.

5.   The method for displaying data according to claim 4, further comprising:

receiving a select operation of the first icon; and
displaying analysis of the association relationship between the living individual and the specified defect based on the select operation of the first icon.

6. The method for displaying data according to any one of claims 1 to 6, wherein the second state diagram is a result of adding a second icon onto the first state diagram, wherein the second icon indicates a processing assessment value of an operation performed by a user.

7. The method for displaying data according to claim 6, wherein a redemption icon is displayed in the second state diagram; and in response to displaying the second state diagram, the method further comprises:

receiving a select operation of the redemption icon;
displaying a redemption page of articles to be redeemed by using the processing assessment value based on the select operation of the redemption icon;
receiving a select operation on the redemption page; and
performing an operation of redeeming an article indicated by the select operation on the redemption page by using the processing assessment value of the user, based on the select operation on the redemption page.

8. The method for displaying data according to any one of claims 1 to 7, wherein an icon for prompting to complete the items to be assessed is further displayed on a display interface of at least one of the first state diagram and the second state diagram.

9. The method for displaying the data according to any one of claims 1 to 8, wherein an icon for prompting to view interaction knowledge related to the items to be assessed is further displayed on the display interface of at least one of the first state diagram and the second state diagram.

10. The method for displaying data according to any one of claims 1 to 9, wherein an assessment icon is displayed on the display interface of at least one of the first state diagram and the second state diagram; and the method further comprises:

receiving a select operation of the assessment icon;
acquiring a score for assessing the vital state of the living individual based on the items to be assessed under instruction of the select operation; and
displaying a third icon on the display interface of the at least one state diagram, wherein the third icon interprets the score.

11. The method for displaying data according to claim 10, further comprising:

receiving a select operation of the third icon; and
displaying score details of the score based on the select operation of the third icon.

12. The method for displaying data according to any one of claims 1 to 11, wherein
prior to displaying the second state diagram, the method further comprises:

assessing the vital state of the living individual based on the items to be assessed; and
displaying the second state diagram comprises:
displaying the second state diagram based on an assessment result of the vital state of the living individual.

13. The method for displaying data according to any one of claims 1 to 11, wherein

prior to displaying the second state diagram, the method further comprises:

sending the items to be assessed to a server; and
receiving an assessment result of the vital state of the living individual sent by the server, wherein the assessment result is acquired based on the items to be assessed; and

displaying the second state diagram comprises:
displaying the second state diagram based on the assessment result of the vital state of the living individual.

14. The method for displaying data according to any one of claims 1 to 13, wherein both the first state diagram and the second state diagram are tree structure diagrams.

15. The method for displaying data according to any one of claims 1 to 14, wherein the vital state of the living individual comprises a health state of the living individual.

16. An apparatus for displaying data, comprising:

a displaying module, configured to display a first state diagram, wherein the first state diagram indicates a vital state of a living individual; and
a receiving module, configured to receive items to be assessed, wherein the items to be assessed are intended to assess the vital state of the living individual;
wherein the displaying module is further configured to display a second state diagram, wherein the second state diagram is a result of updating the first state diagram based on the items to be assessed.

17. The apparatus for displaying data according to claim 16, wherein both the first state diagram and the second state diagram are tree structure diagrams.

18. The apparatus for displaying data according to claim 16 or 17, wherein the vital state of the living individual comprises a health state of a user.

19. A terminal, wherein the terminal comprises a memory, a processor, and a computer program stored on the memory and capable of running on the processor, wherein the program, when run by the processor, causes the processor to perform the method for displaying data as defined in any one of claims 1 to 15.

20. A computer-readable storage medium storing a computer program therein, wherein the program is run to perform the method for displaying data as defined in any one of claims 1 to 15.

Displaying a first state diagram, wherein the first state diagram indicates a vital state of a living individual    S110

Receiving items to be assessed, wherein the items to be assessed are intended to assess the vital state of the living individual    S120

Displaying a second state diagram, wherein the second state diagram is a result of updating the first state diagram based on the items to be assessed    S130

FIG. 1

Health records                    0%

Data    Mall    Me

FIG. 2

## Records

| Monitoring information | Basic information |
| --- | --- |

**Steps**

Today's steps  6,000

**Blood sugar**    4.5 mmol/L in fasting

| <3.9 | 3.9-5.5 | >5.5 |
| --- | --- | --- |
| Too low | Normal | Too high |

**Uric acid**      320 µmol/L in fasting

| <421 | ≥421 |
| --- | --- |
| Normal | Too high |

**Total cholesterol**    3.7 mmol/L in fasting

| <5.2 | ≥5.2 |
| --- | --- |
| Normal | Too high |

◁        ○        □

FIG. 3

## Records

| Monitoring information | Basic information |
| --- | --- |

**Basic information**

Name                                          >
Gender
Date of birth
Height
Weight
Waistline
Marital state

**Disease history**

Hypertension, Diabetes

**Family history**

Father: Hypertension
Mother: Diabetes
Brothers and sisters:
Hypertension and Diabetes
Children: None

**Lifestyle**

Smoking
Drinking

◁        ○        □

FIG. 4

FIG. 5

FIG. 6

**FIG. 7**

Assessment icon

Third icon

99

Health records 60%

Data Mall Me

**FIG. 8**

Score details

Comprehensive score: 99

Risk factor

Knowledge literacy

Health monitoring

Lifestyle

Basic data

FIG. 9

### Diabetes risk assessment

| Assessment result

#### You have a low risk of diabetes at present

Tips: This tool is a diabetes risk assessment model for domestic population recommended by Chinese Diabetes Prevention and Cure Guidelines

Reference: Zhou X, Qiao Q, Ji L, et al. Nonlaboratory-based risk assessment algorithm for undiagnosed type 2 diabetes developed on a nation-wide diabetes survey [J]. Diabetes Care, 2013, 36 (12):3944-52.

80%

80% of users are consistent with your assessment result

| Expert's advice

Recommend you keeping a healthy lifestyle to maintain the low risk:
1. Eat healthy food. Choose foods with low fat, low calorie and high fiber. Focus on fruits, vegetables and whole grains. Try to diversify your foods to prevent boredom.
2. Take more exercise. Aim for 30 minutes of moderate-intensity physical activity every day. Take a walk, ride a bike and swim back and forth every day. If you can't handle longer workouts, break them down throughout the day.
3. Lose excess weight. If you are overweight, losing 7% of your weight can reduce your risk of diabetes. To keep your weight within a healthy range, the key is to permanently change your diet and exercise habits. Encourage yourself by remembering the benefits of losing weight, such as maintaining a healthy heart, gaining more energy and more confidence.

Measure again        Invite friends

Data        Mall        Me

FIG. 10

Items to be
assessed

↓

20≤ age ≤80 —— No

↓ Yes

115 ≤height ≤230
and 32 ≤weight
≤191 —— No

↓ Yes

50≤ systolic
blood pressure ≤140 and
30≤ diastolic blood
pressure ≤90 —— No

↓ Yes

Estimation

↓

End

FIG. 11

Items to be
assessed

↓

Suffering from
diabetes —— Yes

↓ No

Age ≤20 —— Yes

↓ No

Estimation

↓

End

FIG. 12

31

Items to be assessed

↓

45≤ age ≤84 → No

↓ Yes

Estimation

↓

End

FIG. 13

**My points**  Redemption records

**75**

My points

Points redemption

Novice task

First download and registration     +50  ✓

To complete personal health records     +50

Daily task

Health data

≥ 8,000 steps     +20

Electrocardiogram (ECG) measurement (only once a day)     +10

Urine analysis (once a day)     +10

Blood pressure measurement or manual entry (only once a day)     +10

Body fat measurement (only once a day)     +10

| ⌂ | Data | Mall | Me |

FIG. 14

Points redemption | Redemption rule

Course coupon

¥5
Available at over 5 Yuan

Health course coupon

Valid until 90 days from the date of receipt

500 Points

Redeem now

This coupon is for the course and is only used for the course

¥10
Available at over 10 Yuan

Health course coupon

Valid until 90 days from the date of receipt

1000 Points

Redeem now

This coupon is for the course and is only used for the course

¥20
Available at over 20 Yuan

Health course coupon

Valid until 90 days from the date of receipt

2000 Points

Redeem now

This coupon is for the course and is only used for the course

Health course coupon

¥20
Available at over 20 Yuan

Health-care device coupon

Valid until 90 days from the date of receipt

2000 Points

Redeem now

This coupon is for the device and is only used for the device

Data | Mall | Me

FIG. 15

Apparatus for displaying data — 600

Displaying module — 601

Receiving module — 602

FIG. 16

Apparatus for displaying data — 600

Displaying module — 601

Receiving module — 602

Processing module — 603

FIG. 17

600

Apparatus for displaying data

601

Displaying module

602

Receiving module

604

Sending module

FIG. 18

CPU 901

ROM 902

RAM 903

904

Input/Output (I/O) interface 905

Input part 906

Output part 907

Memory portion 908

Communi-cation part 909

Driver 910

Removable medium 911

FIG. 19

<table>
<tr><td colspan="2"><b>INTERNATIONAL SEARCH REPORT</b></td><td>International application No.<br><br><b>PCT/CN2020/131332</b></td></tr>
</table>

**A.    CLASSIFICATION OF SUBJECT MATTER**

G16H 50/30(2018.01)i

According to International Patent Classification (IPC) or to both national classification and IPC

**B.    FIELDS SEARCHED**

Minimum documentation searched (classification system followed by classification symbols)

G16H; G06F

Documentation searched other than minimum documentation to the extent that such documents are included in the fields searched

Electronic data base consulted during the international search (name of data base and, where practicable, search terms used)

WPI, EPODOC, CNPAT, CNKI, IEEE: 虚拟, 形象, 人物, 图形, 更新, 改变, 生命, 评估, 健康, 图标, virtual, image, character, graphics, update, change, life, assessment, health data, icon

**C.    DOCUMENTS CONSIDERED TO BE RELEVANT**

| Category* | Citation of document, with indication, where appropriate, of the relevant passages | Relevant to claim No. |
|---|---|---|
| X | US 2016055758 A1 (FRANCIS, Richard) 25 February 2016 (2016-02-25)<br>description paragraphs [0008], [0010], [0024], [0026], [0031], [0045]-[0047], claim 14, figure 4A | 1-20 |
| X | CN 103781407 A (SAUDI ARABIAN OIL CO. et al.) 07 May 2014 (2014-05-07)<br>description, paragraphs [0021]-[0028] and [0325]-[0330] | 1-20 |
| X | CN 109785967 A (HEBEI WEIYOUQU EDUCATION TECHNOLOGY CO., LTD.) 21 May 2019 (2019-05-21)<br>description, paragraphs [0047]-[0067] | 1-20 |
| A | CN 106845053 A (PKU HEALTHCARE IT CO., LTD.) 13 June 2017 (2017-06-13)<br>entire document | 1-20 |
| A | CN 108847288 A (WUHAN JIULE TECHNOLOGY CO., LTD.) 20 November 2018 (2018-11-20)<br>entire document | 1-20 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **05 February 2021** | **25 February 2021** |

| Name and mailing address of the ISA/CN | Authorized officer |
|---|---|
| **China National Intellectual Property Administration (ISA/ CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

INTERNATIONAL SEARCH REPORT
Information on patent family members

International application No.

**PCT/CN2020/131332**

| Patent document cited in search report | | | Publication date (day/month/year) | Patent family member(s) | | | Publication date (day/month/year) |
|---|---|---|---|---|---|---|---|
| US | 2016055758 | A1 | 25 February 2016 | None | | | |
| CN | 103781407 | A | 07 May 2014 | JP | 2014523040 | A | 08 September 2014 |
| | | | | CA | 2840979 | A1 | 10 January 2013 |
| | | | | AU | 2012279048 | A1 | 23 January 2014 |
| | | | | EP | 2729051 | A2 | 14 May 2014 |
| | | | | WO | 2013006636 | A3 | 21 March 2013 |
| | | | | US | 2013011819 | A1 | 10 January 2013 |
| | | | | US | 2019269369 | A1 | 05 September 2019 |
| CN | 109785967 | A | 21 May 2019 | None | | | |
| CN | 106845053 | A | 13 June 2017 | None | | | |
| CN | 108847288 | A | 20 November 2018 | None | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201911167179